# EUROPEAN PATENT APPLICATION

(11) **EP 4 674 405 A1**
(43) Date of publication of application: **07.01.2026**
(21) Application number: 24185877.8
(22) Date of filing: 02.07.2024
(51) Int. Cl.: A61K 8/64, A61K 8/73, A61K 8/9789, A61Q 19/08

(54) **RENAISSANCE HSP3 - A UNIQUE COMPLEX OF ACTIVE INGREDIENTS FOR SKIN CARE**

(71) Applicant: Hdrey Laboratories Sp. z o.o., 00-710 Warszawa (PL)
(72) Inventor: The designation of the inventor has not yet been filed

(57) **Abstract**

RENAISSANCE HSP3 - a unique complex of active ingredients providing hydration, regeneration, and skin protection. To achieve the best results in improving the appearance and condition of the skin, an active complex of multi-directional ingredients was designed and created.
H - Hyaluronic Acid INCI: Sodium Hyaluronate, Hydrolyzed Hyaluronic Acid
P - Peptide INCI: Acetyl Hexapeptide-8
S - Stem Cells INCI: Haberlea Rhodopensis Leaf Extract

The combination of the well-researched ingredients described above results in a complex that provides:
- Hydration
- Anti-wrinkle
- Regenerative
- Protective
- Antioxidant effects

The use of this complex in cosmetic products for facial and body care will result in safe and highly effective products.

## Description

RENAISSANCE HSP3 - a unique complex of active ingredients providing hydration, regeneration, and skin protection. To achieve the best results in improving the appearance and condition of the skin, an active complex of multi-directional ingredients was designed and created.

### H - Hyaluronic Acid INCI: Sodium Hyaluronate, Hydrolyzed Hyaluronic Acid

These are three hyaluronic acids with different molecular sizes:
1. High Molecular Weight Hyaluronic Acid: The molecular size of high molecular weight hyaluronic acid ranges from 1000 to 1300 kDa. These molecules have t+he ability to retain moisture on the skin's surface, improving its hydration and elasticity. A single application of a product containing this hyaluronic acid enhances skin hydration within one hour. This acid strengthens the skin barrier, creating an exceptional protective layer and giving the skin a fresh effect.
2. Low Molecular Weight Hyaluronic Acid: The smallest hyaluronic acid used in cosmetics, with a molecular weight ranging from 37 to 56 kDa. It is friendly to the microbiome; thanks to its smaller molecules, it can penetrate the stratum corneum, retaining moisture within. This way, it intensively hydrates and smooths fine lines. It improves skin elasticity and makes wrinkles less visible.
3. Medium Molecular Weight Hyaluronic Acid: This hyaluronic acid falls between high and low molecular weight, ranging from 200 to 400 kDa. Thanks to its medium-sized molecules, it can act both on the skin's surface and gently penetrate the skin layers, providing comprehensive hydration, especially when combined with high molecular weight hyaluronic acid. Additionally, it soothes skin redness, improving its appearance.

According to the knowlege described in the literature hyaluronic acid has a broad range of cosmetic and therapeutic applications due to its unique physicochemical properties and involvement in various essential biological processes, including cell signaling, wound reparation, and tissue regeneration. [1] Hyaluronic acid is an important component of the extracellular matrix, with loss starting at 25 years old. Results from several trials generally support that HA-based cosmeceuticals are a non-invasive, effective solution for improving skin hydration, rejuvenation, and healing.[2] Hyaluronic acid is a key molecule associated with skin hydration and its appropriate level in the skin helps alleviate the effects of skin aging.[3]

The combination of the hyaluronic acids described above allows for rapid and long-lasting skin hydration, improves the protective skin barrier, and enhances elasticity, reducing the visibility of wrinkles.

### P - Peptide INCI: Acetyl Hexapeptide-8

A synthetic peptide with anti-wrinkle properties. This peptide was developed to reduce the visibility of fine lines and wrinkles as an alternative to Botox. When applied to the face and neck, Acetyl Hexapeptide-8 helps relax muscles by temporarily limiting the overproduction and release of neurotransmitters that initiate and control the intensity of facial muscle contractions. This leads to a reduction in the visibility of expression wrinkles. Additionally, it weakens the excessive inflammatory response triggered by the IL-33 factor. It prevents damage caused by sleep deprivation, exhibits melatonin-like properties against glycation and peroxidation, and strengthens the first line of immune defense.

According to the knowledge described in the literature, this peptide was tested for its penetration through the epidermis [4], as well as for the relationship between the type of emulsion in which the peptide was used and its penetration.[5] Acetyl Hexapeptide-8 is a bio-safe cosmetic alternative to botulinum toxin which reduces es facial lines and wrinkles. There are many reports confirming the anti-wrinkle effects of this peptide. [6]

### S - Stem Cells INCI: Haberlea Rhodopensis Leaf Extract

Stem cells from the Haberlea Rhodopensis plant, also known as "immortality cells," are a fascinating discovery in skin care. Haberlea Rhodopensis, also known as the flower of Orpheus, grows in harsh conditions such as very low humidity and intense UV radiation, giving it the ability to survive and regenerate. The key aspects related to the action of Haberlea stem cells on the skin include:
1. Regeneration and protection: Stem cells from Haberlea can stimulate skin regeneration and provide protection against harmful environmental factors, such as UV radiation and oxidative stress. They act at the cellular level, supporting the skin's repair and regeneration processes.
2. Hydration and nourishment: Haberlea stem cells also have moisturizing and nourishing properties. They help maintain proper skin hydration levels and provide essential nutrients that can help improve its elasticity and texture.
3. Antioxidant action: Haberlea Rhodopensis is known for its high antioxidant content, which helps combat harmful free radicals. Stem cells from this plant can help neutralize free radical effects on the skin, which may help prevent premature aging and reduce the visibility of wrinkles.
4. Improvement of overall skin condition: Using products containing Haberlea stem cells can lead to an overall improvement in skin condition, including hydration, elasticity, smoothness, and radiance. They act on multiple levels, supporting healthy skin function and preventing damage.

According to the research H. rhodopensis plant extract modulates expression of several crucial stress-responsive genes. It was demonstrated that the Haberlea rhodopensis plant extract, in which myconoside was identified as the most abundant compound, specifically influences the observed dynamics in gene expression.[7] H. rhodopensis plant extract has an inhibitory effect on the inflammatory process occurring as one of the symptoms of hypersensitive skin.[9]

The combination of the well-researched ingredients described above results in a complex that provides:
- Hydration
- Anti-wrinkle
- Regenerative
- Protective
- Antioxidant effects

The use of this complex in cosmetic products for facial and body care will result in safe and highly effective products.

### REFERENCES:

[1] Al-Halaseh LK, Al-Jawabri NA, Tarawneh SK, Al-Qdah WK, Abu-Hajleh MN, Al-Samydai AM, Ahmed MA. "A review of the cosmetic use and potentially therapeutic importance of hyaluronic acid." J Appl Pharm Sci, 2022; 12(07):034-041.
[2] Bruna Bravo, Priscila Correia, José Euzébio Gonçalves Junior, Beatriz Sant'Anna, Delphine Kerob,."Benefits of topical hyaluronic acid for skin quality and signs of skin aging: From literature review to clinical evidence" Dermatologic therapy 35.12 (2022): e15903.
[3] Papakonstantinou, Eleni, Michael Roth, and George Karakiulakis. "Hyaluronic acid: A key molecule in skin aging." Dermato-endocrinology 4.3 (2012): 253-258.
[4] Kraeling, Margaret EK, et al. "In vitro skin penetration of acetyl hexapeptide-8 from a cosmetic formulation." Cutaneous and ocular toxicology 34.1 (2015): 46-52.
[5] Hoppel, Magdalena, et al. "Topical delivery of acetyl hexapeptide-8 from different emulsions: influence of emulsion composition and internal structure." European Journal of Pharmaceutical Sciences 68 (2015): 27-35.
[6] B 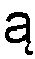 chor, Remigiusz. "Chemical and biological properties of anti-wrinkle peptide Argireline."
[7] Staneva, Dessislava, et al. "Haberlea rhodopensis Extract Tunes the Cellular Response to Stress by Modulating DNA Damage, Redox Components, and Gene Expression." International Journal of Molecular Sciences 24.21 (2023): 15964.
[8] Bankova, Ralitsa. "Haberlea rodopensis-Effects and potential applications." Tradit. Mod. Vet. Med 7 (2022): 128-138.
[9] Kostadinova, Aneliya, et al. "Haberlea rhodopensis extracts affect cell periphery of keratinocytes." Comptes rendus de l'Academie bulgare des Sciences 69.4 (2016).

## Claims

1. qualitative composition of the complex of active ingredients RENAISSANCE HSP3
Hyaluronic Acid (Sodium Hyaluronate, Hydrolyzed Hyaluronic Acid),
Peptide (Acetyl Hexapeptide-8),
Stem Cells (Haberlea Rhodopensis Leaf Extract)

2. quantitative composition of the complex of active ingredients RENAISSANCE HSP3

3. name of the complex of active ingredients RENAISSANCE HSP3
